# EUROPÄISCHE PATENTSCHRIFT

(11) **EP 1 362 051 B1**
(45) Veröffentlichungstag und Bekanntmachung des Hinweises auf die Patenterteilung: **03.08.2005**
(21) Anmeldenummer: 02712902.2
(22) Anmeldetag: 09.02.2002
(51) Int. Cl.: C07D 491/04

(54) **VERFAHREN ZUR REINIGUNG VON 20(S)-CAMPTOTHECIN**
METHOD FOR PURIFYING 20(S)-CAMPTOTHECIN
PROCEDE DE PURIFICATION DE 20(S)-CAMPTOTHECINE

(30) Priorität: 15.02.2001 DE 10106969
(43) Veröffentlichungstag der Anmeldung: 19.11.2003
(73) Patentinhaber: Boehringer Ingelheim Pharma GmbH & Co.KG, 55216 Ingelheim am Rhein (DE)
(72) Erfinder: SOBOTTA, Rainer, 55218 INGELHEIM (DE); RAPP, Armin, 55411 BINGEN-DROMERSHEIM (DE)
(86) Internationale Anmeldenummer: PCT/EP2002/001375
(87) Internationale Veröffentlichungsnummer: WO 2002/064597

(56) Entgegenhaltungen:
- WO-A-94/19353
- IAYAMA R ET AL: "A CAMPTOTHECIN DERIVATIVE FROM NOTHAPODYTES FOETIDA" PHYTOCHEMISTRY, PERGAMON PRESS, GB, Bd. 11, Nr. 27, 1988, Seiten 3663-3664, XP008006380 ISSN: 0031-9422

## Beschreibung

Die Erfindung betrifft ein Reinigungsverfahren von 20(S)-Camptothecin, welches durch ein Vinyl-Camptothecin-Derivat verunreinigt ist.

### Hintergrund der Erfindung

20(S)-Camptothecin (20(S)-CPT) ist ein natürliches Alkaloid der Formel (I) 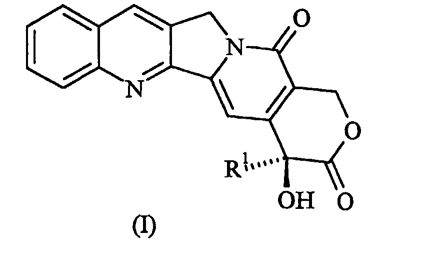 worin R¹ für Ethyl steht.
20(S)-CPT und seine Derivate weisen als Topoisomerase I Inhibitoren Tumor-inhibierende Wirkungen auf (z.B. Giovanelle, B.C. et al., Cancer Research, 51: 302-3055, 1991, European Patent applications EP 0 074 256, EP 0 088 642, US Patents US 4,473,692, US 4,545,880, US 4,604,463 and International Patent application WO 92/05785).
20(S)-CPT kann als Rohprodukt unter anderem von dem chinesischen Baum *Camptotheca acuminata* (Nyssaceae) (Wall M. et al., J. Am. Chem. Soc. 88: 3888-3890, 1966) oder von dem indischen Baum *Nothapodytes foetida* (*nimmoniana*) (frühere Bezeichnung: *Mappie foetida Miers*) (Govindachari, T.R. et al., Phytochemistry 11: 3529-3531, 1972) gewonnen werden.

Diese Rohprodukte, insbesondere das aus *Nothapodytes foetida* gewonnene, weisen 20(S)-CPT verunreingt durch ein CPT-Derivat der Formel (I) auf, worin R¹ für Vinyl steht (20-Vinyl-CPT).

Herkömmlicherweise werden die Rohprodukte durch aufwendige chromatographische Methoden oder durch Überführung des Camptothecins in die wässrige Phase und Entfernen von Verunreinigungen durch Extraktion mit wasserunlöslichen Lösungsmitteln (z.B. WO 94/19353) gereinigt. Die Verunreinigung durch 20-Vinyl-CPT kann allerdings durch diese Methoden nicht effizient entfernt werden.
Es ist daher Aufgabe der vorliegenden Erfindung, ein Verfahren bereitzustellen, das die Aufreinigung des 20(S)-CPT Ausgangproduktes ohne Anwendung aufwendiger chromatographischer Methoden erlaubt.

### Beschreibung der Erfindung

Überraschenderweise wurde gefunden, dass 20(S)-CPT nahezu vollständig von Verunreinigungen durch 20-Vinyl-CPT befreit werden kann, indem man das Ausgangsmaterial zuerst mit einer wässrigen Base behandelt, hydriert und anschließend ansäuert und das Produkt isoliert.

Gegenstand der Erfindung ist somit ein Verfahren zur Reinigung von 20(S)-Camptothecin, welches folgende Schritte umfasst:
(a) Zusammengeben einer wässrigen Base und eines 20(S)-Camptothecin enthaltenden Ausgangsmaterial, wobei der Lactonring des 20(S)-Camptothecins zu einem Carboxylatsalz umgewandelt wird;
(b) Hydrierung des erhaltenen Gemischs in Gegenwart eines Übergangmetallkatalysators;
(c) Ansäuern der wässrigen Phase unter Bildung von Camptothecinkristallen;
(d) Zusatz eines polar aprotischen Lösungsmittels; und
(e) Abtrennen der Camptothecinkristalle.

Ein weiterer Gegenstand der Erfindung ist ein Verfahren zur Herstellung von 20(S)-Camptothecin der Formel (I), worin R¹ Ethyl bedeutet, aus 20-Vinyl-Camptothecin der Formel (I), worin R¹ Vinyl bedeutet, welches folgende Schritte umfasst:
(a) Zusammengeben einer wässrigen Base und des 20-Vinyl-Camptothecin enthaltenden Ausgangsmaterials, unter Bildung einer Verbindung der Formel (II), 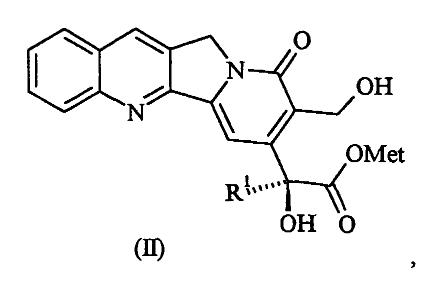 worin
   R¹ für Vinyl steht; und
   Met für ein Metall steht;
(b) Hydrierung des erhaltenen Gemischs in Gegenwart eines Übergangmetallkatalysators;
(c) Ansäuern der wässrigen Phase unter Bildung von Camptothecinkristallen;
(d) Zusatz mindestens eines polar aprotischen Lösungsmittels; und
(e) Abtrennen der Camptothecinkristallen.

### Detaillierte Beschreibung der Erfindung

Der Begriff "Camptothecin enthaltendes Ausgangsmaterial" wie er vor und nachstehend benutzt wird umfasst ein verunreinigtes Material enthaltend 20(S)-CPT, rohes Camptothecin, Camptothecin-haltige Pflanzenextrakte, synthetisches Camptothecin Derivate des Camptothecins wie sie zum Beispiel in der Internationalen Patentanmeldung WO 92/05785 beschrieben sind, oder Reaktionsprodukte enthaltend Camptothecin.

Vorzugsweise ist das Ausgangsmateial ein natürliches Rohprodukt, welches insbesondere von *Nothapodytes foetida* gewonnen wurde. In der Regel ist es ein Gemisch aus der Verbindung der Formel (I), worin R¹ für Ethyl steht, und der Verbindung der Formel (I), worin R¹ für Vinyl steht. Es enthält in der Regel 0,9 bis 1,5 Gew.-%, vorzugsweise 1,0 bis 1,4 Gew-% der Vinyl-Verbindung. Daneben kann das Ausgangsmaterial noch andere Camptothecin-Derivate enthalten wie zum Beispiel 9-Methoxy-CPT, 10-Methoxy-CPT, 11-Methoxy-CPT, 10-Hydroxy-CPT und 11-Hydroxy-CPT. Das Ausgangsmaterial enthält sind in der Regel bis zu 1 Gew-% an einem oder mehreren dieser zusätzlichen CPT-Derivate, insbesondere 0,2 bis 0,8 Gew.-% 9-Methoxy-CPT.

Der Begriff "wässrige Base" wie er vor und nachstehend bezüglich des Schrittes (a) des erfindungsgemäßen Reinigungsverfahrens verwendet wird, betrifft eine Base, die im wässrigen Medium, vorzugsweise in reinem Wasser, genügend Hydroxidionen erzeugt, um die Laktongruppe der im Ausgangsmaterial enthaltenen Camptothecin-Derivate vollständig in die entsprechenden Hydroxycarboxylate zu überführen. Bevorzugt sind Metallhydroxide, insbesondere Alkalimetall- oder Erdalkalimetallhydroxide wie Lithiumhydroxid, Natriumhydroxid, Kaliumhydroxid oder Kalziumhydroxid. Am meisten bevorzugt ist Natriumhydroxid.

Das Metallhydroxid wird vorzugsweise in Form einer verdünnten wässrigen Lösung eingesetzt, vorzugsweise in Form einer 1 bis 25 %-igen, insbesondere 3 bis 10 %-gen wässrigen Lösung. In der Regel wird soviel Metallhydroxid eingesetzt, dass die Camptothecin-Derivate vollständig in Lösung gehen; vorzugsweise werden 1 bis 20 mol, besonders vorzugsweise 5 bis 15 mol, insbesondere 7,5 bis 12,5 mol Metallhydroxid bezogen auf 1 mol Ausgangsmaterial eingesetzt.

Zu dem erhaltenen Gemisch wird in Schritt (b) mit einem Übergangsmetallkatalysator, vorzugsweise einem heterogenen Übergangsmetallkatalysator, insbesondere Platin, Platinoxid, Nickel, Palladium oder Rhodium auf einem Trägermaterial wie Aktivkohle oder Aluminiumoxid hinzugefügt. Bevorzugt ist Palladium auf Aktivkohle mit einem Gehalt von 1 bis 15 Gew.%, vorzugsweise 2 bis 10 Gew.-%, insbesondere etwa 5 Gew.-% Palladium.

Die Menge an Übergangsmetallkatalysator wird so gewählt, dass eine vollständige Hydrierung des vinylischen CPT-Derivates gewährleistet ist. Vorzugsweise werden 0,01 bis 0,50 Gewichtsteile, insbesondere 0,02 bis 0,10 Gewichtsteile Übergangsmetallkatalysator (inklusive Trägermaterial) bezogen auf 1 Gewichtsteil des Ausgangsmaterials eingesetzt.

Da so erhaltene Gemisch wird vorzugsweise bei einer Temperatur von - 20 °C bis 100 °C, insbesondere von 10 °C bis 40 °C, meist bevorzugt etwa bei Raumtemperatur, mit gasförmigem Wasserstoff beaufschlagt.

Der Wasserstoffdruck ist an sich unkritisch, vorzugsweise wird die Hydrierung bei Normaldruck oder leichtem Überdruck, insbesondere bei 0,9 bis 5,0 bar, meist bevorzugt bei etwa 1 bar durchgeführt.

Unter den angegebenen ist die Hydrierung in der Regel innerhalb von 1 bis 20 Stunden, vorzugsweise 4 bis 15 Stunden, insbesondere 6 bis 10 Stunden vollständig.

Nach erfolgter Hydrierung wird der Übergangsmetallkatalysator vorzugsweise durch Filtration entfernt, und die erhaltene Reaktionsmischung wird in Schritt (c) angesäuert. Das Ansäuern kann mit einer anorganischen oder organischen Säure erfolgen. Bevorzugte Säuren sind Mineralsäuren wie HCl, HBr, HJ, HNO₃, H₃PO₄, H₂SO₄, oder aliphatische Carbonsäuren wie Essigsäure und Trifluoressigsäure oder Mischungen dieser Säuren, insbesondere konzentrierte Salzsäure. Mit der jeweiligen Säure wird ein pH-Wert von 3,0 bis 6,0, vorzugsweise 3,5 bis 5,0, insbesondere von etwa 4,0 bis 4,5 eingestellt. Die Umsetzung mit der Säure erfolgt in der Regel bei einer Temperatur von 0 °C bis 100 °C, vorzugsweise von 30 °C bis 80 °C, insbesondere von 50 °C bis 60 °C.

In einer besonders bevorzugten Ausführungsform wird mit 2 bis 20 Gewichtsteilen, vorzugsweise 4 bis 9 Gewichtsteilen, insbesondere 6 bis 8 Gewichtsteilen konzentrierter Salzsäure bezogen auf 1 Gewichtsteil Ausgangsmaterial angesäuert.

Unter den angegebenen Bedingungen ist die Lactonisierung zum CPT in der Regel innerhalb von 10 bis 180 Minuten, vorzugsweise 15 bis 60 Stunden, insbesondere innerhalb von etwa 30 Minuten vollständig.

Das durch das Ansäuern erhaltene Reaktionsgemisch fällt in der Regel als reine Suspension an. Zur Verbesserung der Kristallisation in Schritt (d) wird diese mit einem oder mehreren polar aprotischen Lösungsmitteln versetzt. Als solche eignen sich vorzugsweise Sulfoxide wie zum Beispiel Dimethylsulfoxid (DMSO) oder Amide und Harnstoff-Derivate der Formel 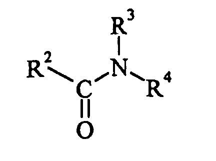 worin
R² für Wasserstoff oder eine C₁-C₄ Alkylgruppe, insbesondere Wasserstoff oder Methyl steht;
R³ und R⁴ jeweils unabhängig voneinander für eine C₁-C₄ Alkylgruppe, insbesondere Methyl stehen; oder
R² und R³ zusammen für eine -(CH₂)ₘ- oder eine -NR⁵-(CH₂)ₙ- Gruppe stehen, wobei
R⁵ für eine C₁-C₄ Alkylgruppe steht;
m 3 oder 4, insbesondere 3 ist; und
n 2 oder 3 ist,
insbesondere ausgewählt aus der Gruppe bestehend aus N,N-Dimethylformamid (DMF), N,N-Dimethylacetamid (DMA), N-Methylpyrrolidon (NMP), N,N-Dimethylethylenharnstoff (DMEU) und N,N-Dimethylpropylenharnstoff (DMPU) oder Mischungen dieser Lösungsmittel, meist bevorzugt DMF.

In der Regel werden 10 bis 100 Gewichtsteile, vorzugsweise, 20 bis 80 Gewichtsteile, insbesondere 30 bis 50 Gewichtsteile des polar aprotischen Lösungsmittels bezogen auf 1 Gewichtsteil des eingesetzten Ausgangsmaterials verwendet.

Die Behandlung mit dem polar aprotischen Lösungsmittel kann bei jeder beliebigen Temperatur durchgeführt werden. Das Reaktionsgemisch wird vorzugsweise bei einer Temperatur von 30 °C bis 120 °C, insbesondere von 80 bis 100 °C gerührt und anschließend langsam auf Raumtemperatur abgekühlt.

Die so erhaltenen CPT-Kristalle können in Schritt (e) leicht von der flüssigen Phase abgetrennt werden, vorzugsweise erfolgt die Abtrennung durch Dekantieren, Zentrifugieren, Schleudern, Abpressen oder Filtration, insbesondere durch Filtration.

In der Regel werden die erhaltenen CPT Kristalle mit einem Alkohol, vorzugsweise Methanol, Ethanol oder Iosopropanol, insbesondere Methanol gewaschen und getrocknet.

Der Vorteil der erfindungsgemäßen Vorgehensweise ist die hohe Raum-Zeit-Ausbeute sowie die hohe Ausbeute und Reinheit des erhaltenen 20(S)-Camptothecins, welches ohne chromatographische Aufreinigung im wesentlichen frei von Vinylgruppen-haltigen Verunreinigungen erhalten wird.

Die nachfolgenden Beispiele dienen der Illustration exemplarisch durchgeführter Verfahren zur Reinigung der Camptothecins. Sie sind lediglich als mögliche, exemplarisch dargestellte Vorgehensweisen zu verstehen, ohne die Erfindung auf deren Inhalt zu beschränken.

### Beispiel 1

10,45 g eines Camptothecin haltigen, aus *Nothapodytes foetida* gewonnenen Rohproduktes enthaltend 1,33 % 20-Vinyl-CPT und 0,47 % 9-Methoxy-CPT wird in 260 ml einer 2 normalen Natronlauge aufgenommen und mit 0,6 g Palladium/Aktivkohle (5 %-ig) versetzt. Das Gemisch wird 8 Stunden bei Raumtemperatur und einem Druck von 1 bar mit Wasserstoff beaufschlagt.

Anschließend wird die Reaktionsmischung filtriert und bei 50-60 °C mit 80 ml konzentrierter Salzsäure versetzt und auf einen pH-Wert von 4,0 bis 4,5 eingestellt.

Die entstandene Suspension wird mit 400 ml DMF versetzt und 2,5 Stunden bei 90-100 °C gerührt. Die erhaltene Suspension wird langsam auf Raumtemperatur abgekühlt und filtriert. Die gewonnenen CPT-Kristalle werden mit 100 ml Methanol gewaschen und bei 55 °C *in vacuo* getrocknet.

Man erhält 9,85 g (94,2 % des Einsatzes) an 20(S)-Camptothecin mit einem Gehalt von weniger als 0,05 % 20-Vinyl-CPT und 0,11 % 9-Methoxy CPT.

### Beispiel 2

10,45 g eines Camptothecin haltigen, aus *Nothapodytes foetida* gewonnenen Rohproduktes enthaltend 1,33 % 20-Vinyl-CPT und 0,47 % 9-Methoxy-CPT wird in 260 ml einer 2 normalen Natronlauge aufgenommen und mit 0,6 g Palladium/Aktivkohle (5 %-ig) versetzt. Das Gemisch wird 8 Stunden bei Raumtemperatur und einem Druck von 1 bar mit Wasserstoff beaufschlagt.

Anschließend wird die Reaktionsmischung filtriert und bei 50-60 °C mit 300 ml einer 10 %-igen Schwefelsäure versetzt und auf einen pH-Wert von 4,0 bis 4,5 eingestellt.

Die entstandene Suspension wird mit 500 ml DMF versetzt und 2,5 Stunden bei 90-100 °C gerührt. Die erhaltene Suspension wird langsam auf Raumtemperatur abgekühlt und filtriert. Die gewonnenen CPT-Kristalle werden mit 100 ml Methanol gewaschen und bei 55 °C *in vacuo* getrocknet.

Man erhält 9,67 g (92,6 % des Einsatzes) an 20(S)-Camptothecin mit einem Gehalt von 0,09 % 9-Methoxy CPT, wobei der Gehalt an 20-Vinyl-CPT unterhalb der Nachweisgrenze liegt.

## Patentansprüche

1. Verfahren zur Reinigung von 20(S)-Camptothecin, welches folgende Schritte umfasst:
(a) Zusammengeben einer wässrigen Base und eines 20(S)-Camptothecin enthaltenden Ausgangsmaterial, wobei der Lactonring des 20(S)-Camptothecins zu einem Carboxylatsalz umgewandelt wird;
(b) Hydrierung des erhaltenen Gemischs in Gegenwart eines Übergangmetallkatalysators;
(c) Ansäuern der wässrigen Phase unter Bildung von Camptothecinkristallen;
(d) Zusatz mindestens eines polar aprotischen Lösungsmittels; und
(e) Abtrennen der Camptothecinkristallen.

2. Verfahren zur Reinigung von 20(S)-Camptothecin nach Anspruch 1, **dadurch gekennzeichnet, dass** das 20(S)-Camptothecin enthaltende Ausgangsmaterial ein natürliches Pflanzenprodukt ist.

3. Verfahren zur Reinigung von 20(S)-Camptothecin nach Anspruch 1 oder 2, **dadurch gekennzeichnet, dass** das 20(S)-Camptothecin enthaltende Ausgangsmaterial im wesentlichen aus einem Gemisch der Verbindungen der Formel (I) besteht, 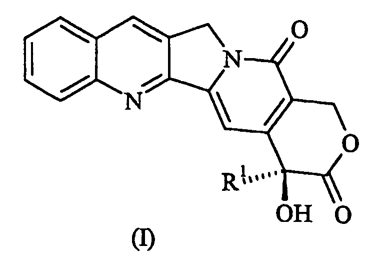 worin R¹ für Ethyl oder Vinyl steht.

4. Verfahren zur Reinigung von 20(S)-Camptothecin nach einem der vorangehenden Ansprüche, **dadurch gekennzeichnet, dass** die Base in Schritt (a) Natriumhydroxid ist.

5. Verfahren zur Reinigung von 20(S)-Camptothecin nach einem der vorangehenden Ansprüche, **dadurch gekennzeichnet, dass** man das in Schritt (a) erhaltene Gemisch in Gegenwart eines Palladium-Katalysators bei einer Temperatur von 0 °C bis 100 °C und beim einem Druck von 0,5 bar bis 5,0 bar hydriert.

6. Verfahren zur Reinigung von 20(S)-Camptothecin nach einem der vorangehenden Ansprüche, **dadurch gekennzeichnet, dass** man die in Schritt (b) erhaltene wässrige Phase mit einer Säure ausgewählt aus der Gruppe HCl, HBr, HJ, HNO₃, H₃PO₄, H₂SO₄, Essigsäure und Trifluoressigsäure oder Mischungen dieser Säuren bei einer Temperatur von 30 °C bis 80 °C behandelt.

7. Verfahren zur Reinigung von 20(S)-Camptothecin nach einem der vorangehenden Ansprüche, **dadurch gekennzeichnet, dass** man die in Schritt (c) erhaltene wässrige Phase mit einem oder mehreren polar aprotischen Lösungsmitteln der Formel 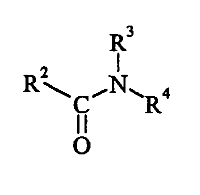 worin
R² für Wasserstoff oder eine C₁-C₄ Alkylgruppe steht;
R³ und R⁴ jeweils unabhängig voneinander für eine C₁-C₄ Alkylgruppe stehen; oder
R² und R³ zusammen für eine -(CH₂)ₘ- oder eine -NR⁵-(CH₂)ₙ- Gruppe stehen, wobei
R⁵ für eine C₁-C₄ Alkylgruppe steht;
m 3 oder 4 ist; und
n 2 oder 3 ist,
bei einer Temperatur von 30 °C bis 120 °C behandelt.

8. Verfahren zur Reinigung von 20(S)-Camptothecin nach Anspruch 7, **dadurch gekennzeichnet, dass** das polar aprotische Lösungsmittel ausgewält ist aus der Gruppe bestehend aus N,N-Dimethylformamid (DMF), N,N-Dimethylacetamid (DMA), N-Methylpyrrolidon (NMP), N,N-Dimethylethylenharnstoff (DMEU) und N,N-Dimethylpropylenharnstoff (DMPU) oder Mischungen dieser Lösungsmittel.

9. Verfahren zur Reinigung von 20(S)-Camptothecin nach einem der vorangehenden Ansprüche, **dadurch gekennzeichnet, dass** die in Schritt (d) 20(S)-Camptothecinkristalle durch Filtration abgetrennt werden.

10. Verfahren zur Herstellung von 20(S)-Camptothecin der Formel (I), worin R¹ Ethyl bedeutet, aus einem 20-Vinyl-Camptothecin der Formel (I), worin R¹ Vinyl bedeutet, welches folgende Schritte umfasst:
(a) Zusammengeben einer wässrigen Base und des 20-Vinyl-Camptothecin enthaltenden Ausgangsmaterials, unter Bildung einer Verbindung der Formel (II), 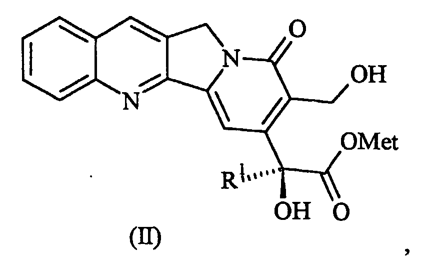 worin
R¹ für Vinyl steht; und
Met für ein Metall steht;
(b) Hydrierung des erhaltenen Gemischs in Gegenwart eines Übergangmetallkatalysators;
(c) Ansäuern der wässrigen Phase unter Bildung von Camptothecinkristallen;
(d) Zusatz mindestens eines polar aprotischen Lösungsmittels; und
(e) Abtrennen der Camptothecinkristallen.

## Claims

1. Process for purifying 20(S)-camptothecine, which comprises the following steps:
(a) combining an aqueous base and a starting material containing 20(S)-camptothecine, thereby converting the lactone ring of the 20(S)-camptothecine into a carboxylate salt;
(b) hydrogenating the resulting mixture in the presence of a transition metal catalyst;
(c) acidifying the aqueous phase, thereby forming camptothecine crystals;
(d) adding at least one polar aprotic solvent; and
(e) separating off the camptothecine crystals.

2. Process for purifying 20(S)-camptothecine according to claim 1, **characterised in that** the starting material containing 20(S)-camptothecine is a natural plant product.

3. Process for purifying 20(S)-camptothecine according to claim 1 or 2, **characterised in that** the starting material containing 20(S)-camptothecine consists essentially of a mixture of the compounds of formula (I), 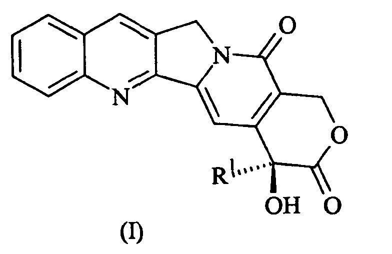 wherein R¹ denotes ethyl or vinyl.

4. Process for purifying 20(S)-camptothecine according to one of the preceding claims, **characterised in that** the base in step (a) is sodium hydroxide.

5. Process for purifying 20(S)-camptothecine according to one of the preceding claims, **characterised in that** the mixture obtained in step (a) is hydrogenated in the presence of a palladium catalyst at a temperature of 0 °C to 100 °C and at a pressure of 0.5 bar to 5.0 bar.

6. Process for purifying 20(S)-camptothecine according to one of the preceding claims, **characterised in that** the aqueous phase obtained in step (b) is treated with an acid selected from among HCl, HBr, HI, HNO₃, H₃PO₄, H₂SO₄, acetic acid and trifluoroacetic acid or mixtures of these acids at a temperature of 30 °C to 80 °C.

7. Process for purifying 20(S)-camptothecine according to one of the preceding claims, **characterised in that** the aqueous phase obtained in step (c) is treated with one or more polar aprotic solvents of formula 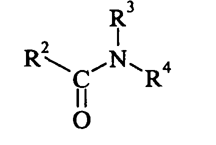 wherein
R² denotes hydrogen or a C₁₋₄ alkyl group;
R³ and R⁴ independently of each other denote a C₁₋₄ alkyl group; or
R² and R³ together denote a -(CH₂)ₘ- or a -NR⁵-(CH₂)ₙ- group, while
R⁵ denotes a C₁₋₄ alkyl group;
m is 3 or 4; and
n is 2 or 3,
at a temperature of 30 °C to 120 °C.

8. Process for purifying 20(S)-camptothecine according to claim 7, **characterised in that** the polar aprotic solvent is selected from among N,N-dimethylformamide (DMF), N,N-dimethylacetamide (DMA), N-methylpyrrolidone (NMP), N,N-dimethylethylene urea (DMEU) and N,N-dimethylpropylene urea (DMPU) or mixtures of these solvents.

9. Process for purifying 20(S)-camptothecine according to one of the preceding claims, **characterised in that** the 20(S)-camptothecine crystals in step (d) are separated off by filtration.

10. Process for preparing 20(S)-camptothecine of formula (I) wherein R¹ denotes ethyl from a 20-vinyl-camptothecine of formula (I) wherein R¹ denotes vinyl, which comprises the following steps:
(a) combining an aqueous base and the starting material containing 20-vinyl-camptothecine, to form a compound of formula (II), 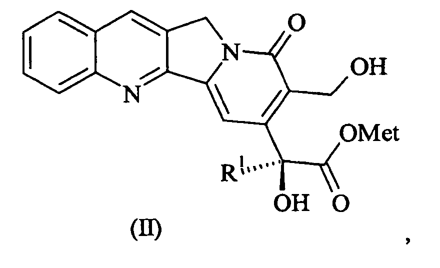 wherein
R¹ denotes vinyl; and
Met denotes a metal;
(b) hydrogenating the resulting mixture in the presence of a transition metal catalyst;
(c) acidifying the aqueous phase to form camptothecine crystals;
(d) adding at least one polar aprotic solvent; and
(e) separating off the camptothecine crystals.

## Revendications

1. Procédé de purification de 20(S)-camptothécine qui comprend les étapes suivantes :
(a) la réunion d'une base aqueuse et d'un produit de départ contenant de la 20(S)-camptothécine, où le cycle lactone de la 20(S)-camptothécine est converti en un sel carboxylate ;
(b) l'hydrogénation du mélange obtenu en présence d'un catalyseur de métal de transition ;
(c) l'acidification de la phase aqueuse avec formation de cristaux de camptothécine ;
(d) l'addition d'au moins un solvant aprotique polaire ; et
(e) la séparation des cristaux de camptothécine.

2. Procédé de purification de 20(S)-camptothécine selon la revendication 1 **caractérisé en ce que** le produit de départ contenant de la 20(S)-camptothécine est un produit végétal naturel.

3. Procédé de purification de 20(S)-camptothécine selon la revendication 1 ou 2 **caractérisé en ce que** le produit de départ contenant de la 20(S)-camptothécine consiste essentiellement en un mélange des composés de formule (I) 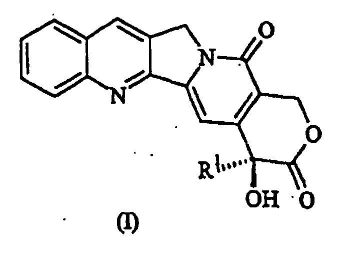 où R¹ représente éthyle ou vinyle.

4. Procédé de purification de 20(S)-camptothécine selon l'une des revendications précédentes **caractérisé en ce que** la base dans l'étape (a) est l'hydroxyde de sodium.

5. Procédé de purification de 20(S)-camptothécine selon l'une des revendications précédentes **caractérisé en ce que** l'on hydrogène le mélange obtenu dans l'étape (a) en présence d'un catalyseur au palladium à une température de 0°C à 100°C et à une pression de 0,5 bar à 5,0 bar.

6. Procédé de purification de 20(S)-camptothécine selon l'une des revendications précédentes **caractérisé en ce que** l'on traite la phase aqueuse obtenue dans l'étape (b) avec un acide choisi dans le groupe HCl, HBr, HI, HNO₃, H₃PO₄, H₂SO₄, acide acétique et acide trifluoroacétique ou des mélanges de ces acides à une température de 30°C à 80°C.

7. Procédé de purification de 20(S)-camptothécine selon l'une des revendications précédentes **caractérisé en ce que** l'on traite la phase aqueuse obtenue dans l'étape (c) avec un ou plusieurs solvants aprotiques polaires de formule 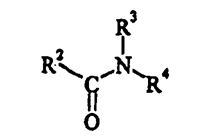 où
R² représente l'hydrogène ou un groupe C₁-C₄ alkyle ;
R³ et R⁴ représentent chacun indépendamment l'un de l'autre un groupe C₁-C₄ alkyle ; ou
R² et R³ représentent ensemble un groupe -(CH₂)ₘ- ou un groupe -NR⁵-(CH₂)ₙ-, où
R⁵ représente un groupe C₁-C₄ alkyle ;
m est 3 ou 4 ; et
n est 2 ou 3,
à une température de 30°C à 120°C.

8. Procédé de purification de 20(S)-camptothécine selon la revendication 7 **caractérisé en ce que** le solvant aprotique polaire est choisi dans le groupe consistant en le N,N-diméthylformamide (DMF), le N,N-diméthylacétamide (DMA), la N-méthylpyrrolidone (NMP), la N,N-diméthyléthylèneurée (DMEU) et la N,N-diméthylpropylèneurée (DMPU) ou des mélanges de ces solvants.

9. Procédé de purification de 20(S)-camptothécine selon l'une des revendications précédentes **caractérisé en ce que** les cristaux de 20(S)-camptothécine dans l'étape (d) sont séparés par filtration.

10. Procédé de production de 20(S)-camptothécine de formule (I) où R¹ représente éthyle, à partir d'une 20-vinyl-camptothécine de formule (I) où R¹ représente vinyle, qui comprend les étapes suivantes :
(a) la réunion d'une base aqueuse et du produit de départ contenant de la 20-vinyl-camptothécine, avec formation d'un composé de formule (II) : 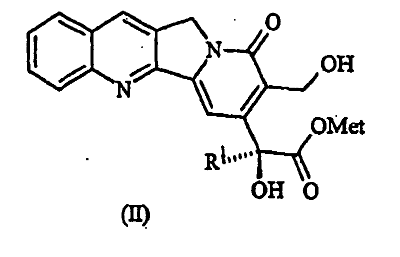 où
R¹ représente vinyle ; et
Met représente un métal ;
(b) l'hydrogénation du mélange obtenu en présence d'un catalyseur de métal de transition ;
(c) l'acidification de la phase aqueuse avec formation de cristaux de camptothécine ;
(d) l'addition d'au moins un solvant aprotique polaire ; et
(e) la séparation des cristaux de camptothécine.
